## Europäisches Patentamt
## European Patent Office
## Office européen des brevets

(19)

(11) Veröffentlichungsnummer: **0 170 182 B1**

(12) **EUROPÄISCHE PATENTSCHRIFT**

(45) Veröffentlichungstag der Patentschrift: **15.05.91**

(51) Int. Cl.⁵: **C07C 11/08**, C07C 5/25, C07C 7/04

(21) Anmeldenummer: 85109124.9

(22) Anmeldetag: 22.07.85

(54) Verfahren zur Gewinnung von 2-Butenen aus 1-Buten und gegebenenfalls 2-Butene enthaltenden C4-Kohlenwasserstoffgemischen.

(30) Priorität: 28.07.84 DE 3427979

(43) Veröffentlichungstag der Anmeldung:
05.02.86 Patentblatt 86/06

(45) Bekanntmachung des Hinweises auf die Patenterteilung:
15.05.91 Patentblatt 91/20

(84) Benannte Vertragsstaaten:
AT BE DE FR GB IT NL SE

(56) Entgegenhaltungen:
EP-A- 0 129 900
FR-A- 2 130 387

(73) Patentinhaber: **BASF Aktiengesellschaft**
**Carl-Bosch-Strasse 38**
**W-6700 Ludwigshafen(DE)**

(72) Erfinder: **Lindner, Alfred, Dr.**
**Ringstrasse 30**
**W-6712 Bobenheim-Roxheim(DE)**
Erfinder: **Mross, Wolf Dieter, Dr.**
**Anselm-Feuerbach-Strasse 21**
**W-6710 Frankenthal(DE)**
Erfinder: **Strohmeyer, Max, Dr.**
**Woogstrasse 41**
**W-6703 Limburgerhof(DE)**
Erfinder: **Volkamer, Klaus, Dr.**
**Heidelberger Ring 21**
**W-6710 Frankenthal(DE)**
Erfinder: **Die anderen Erfinder haben auf ihre Nennung verzichtet**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft ein Verfahren zur Gewinnung von 2-Butenen aus 1-Buten, n-Butan und gegebenenfalls 2-Butene enthaltenden $C_4$-Kohlenwasserstoffgemischen durch katalytische Isomerisierung des 1-Butens zu 2-Butenen.

Es ist bereits bekannt, z.B. aus der DE-OS 30 22 821 und EP-A-83 705, 1-Buten katalytisch zu 2-Butenen zu isomerisieren. Die bekannten Verfahren haben jedoch den Nachteil, daß entweder die Produktreinheit nicht ausreichend ist, Oligomere gebildet werden, die Ausbeute unbefriedigend ist oder der Katalysator nur schwierig zu regenerieren ist.

Es wurde nun ein vorteilhaftes Verfahren gefunden zur Gewinnung von 2-Butenen aus 1-Buten, n-Butan und gegebenenfalls 2-Butene enthaltenden $C_4$-Kohlenwasserstoffgemischen, bei dem man das 1-Buten, n-Butan und gegebenenfalls 2-Butene enthaltende $C_4$-Kohlenwasserstoffgemisch einer Isomerisierungszone (5) zuführt (1, 2, 4), in der Isomerisierungszone das 1-Buten bei erhöhten Temperaturen zu 2-Butenen isomerisiert, das aus der Isomerisierungszone erhaltene Isomerisierungsgemisch in das untere oder mittlere Drittel der destillativen Trennzone (3) leitet (7), oberhalb des unteren Drittels der destillativen Trennzone eine 1-Buten und zusätzlich 2-Buten-trans enthaltende Fraktion abzieht (9) und in die Isomerisierungszone leitet (2, 4), im unteren Drittel der destillativen Trennzone die 2-Butene bzw. eine die 2-Butene enthaltende Fraktion abzieht (8) und gleichzeitig eine n-Butan enthaltende Fraktion aus der destillativen Trennzone an einem Punkt oberhalb des unteren Drittels der destillativen Trennzone abzieht (12).

Nach dem erfindungsgemäßen Verfahren wird 2-Buten-Produkt in guter Ausbeute und hinreichender Reinheit erhalten, wobei die Reinheit je nach Verwendungszweck einstellbar ist. Es ist ein besonderer Vorteil des Verfahrens, daß eine aufwendige Extraktivdestillation und die damit verbundene Zufuhr von Fremdstoffen nicht erforderlich ist. Der spezifische Energieverbrauch läßt sich niedrig halten durch Nutzung der aus der Isomerisierung erhaltenenWärme z.B. zur Vorheizung des in die Isomerisierung eingeleiteten Ausgangs-$C_4$-Kohlenwasserstoffgemisches oder für die Aufkochung der Destillation. Das niedrige Temperaturniveau ermöglicht zudem die Nutzung von wohlfeiler Abwärme.

Die für das erfindungsgemäße Verfahren einzusetzenden 1-Buten, n-Butan und gegebenenfalls 2-Butene enthaltenden $C_4$-Kohlenwasserstoffgemische weisen im allgemeinen einen Gehalt an 1-Buten und gegebenenfalls 2-Butenen von 70 bis 90 Gew.% auf. 2-Buten kann als 2-Buten-cis oder 2-Buten-trans enthalten sein. In der Regel ist das gegebenenfalls im Ausgangs-$C_4$-Kohlenwasserstoffgemisch enthaltene 2-Buten jedoch ein Gemisch aus 2-Buten-cis und 2-Buten-trans. Neben dem 1-Buten kann das Ausgangs-$C_4$-Kohlenwasserstoffgemisch bis zu 90 Gew.%, in der Regel 1 bis 70 Gew.%, insbesondere 1 bis 60 Gew.% 2-Butene enthalten. Neben 1-Buten, n-Butan und gegebenenfalls 2-Butenen können die Ausgangs-$C_4$-Kohlenwasserstoffgemische noch weitere $C_4$-Kohlenwasserstoffe wie Isobutan, Isobuten, enthalten.

Durch die erfindungsgemäße Arbeitsweise ist es möglich, ein 2-Buten-Produkt zu erhalten, das stark an n-Butan abgereichert ist, obwohl sich die Siedepunkte von n-Butan und 2-Buten-trans nur geringfügig unterscheiden. Im allgemeinen wird man eine solche Menge an 2-Buten-trans in die Isomerisierungszone zurückführen, daß das Gewichtsverhältnis von in die Isomerisierungszone zurückgeführtem 2-Buten-trans zu dem abgezogenen n-Butan zweckmäßig 100:1 bis 1:100, vorzugsweise 50:1 bis 1:50, insbesondere 10:1 bis 1:10 beträgt.

Für die Isomerisierung können eine oder mehrere Isomerisierungsstufen verwendet werden. Im allgemeinen wird man eine Isomerisierungsstufe anwenden. Bei Gewinnung von Butan-armem Buten-2-Produkt kann es jedoch zweckmäßig sein, mehrere Isomerisierungsstufen, z.B. 2 oder 3 Stufen, zu verwenden.

Als Katalysator für die Isomerisierung der 2-Butene zu 1-Buten werden zweckmäßig saure Katalysatoren verwendet. Die sauren Katalysatoren können in flüssiger Phase, z.B. als wäßrige Mineralsäuren, vorzugsweise wäßrige Phosphorsäuren, z.B. Phosphorsäuren mit einem Gehalt an $P_2O_5$ von 1 bis 80 Gew.%, vorzugsweise 2 bis 75 Gew.%, oder als feste Katalysatoren verwendet werden.

Als feste saure Katalysatoren sind z.B. feste Phosphorsäurekatalysatoren, die Mono- oder vorzugsweise Polyphosphorsäure auf einem festen Trägermaterial enthalten, vorteilhaft geeignet. Geeignete Trägermaterialien für die Phosphorsäurekatalysatoren sind z.B. Aluminiumoxid, Kieselsäure, Aktivkohle, Kieselgur oder Bims. Vorzugsweise wird Kieselgel als Trägermaterial verwendet.

Weitere geeignete saure Katalysatoren sind saure Metallsulfate wie Natriumhydrogensulfat, Calciumhydrogensulfat, Aluminiumsulfate, Nickelsulfat, Kupfersulfat, Kobaltsulfat, Cadmiumsulfat, Strontiumsulfat. Diese sauren Metallsulfate können als solche verwendet werden. Vorzugsweise werden sie auf einem Trägermaterial angewendet. Geeignete Trägermaterialien sind z.B. Kieselgel, Aktivkohle, Aluminiumoxid oder Bims.

Weiter kommen Kieselgel oder Aluminiumoxid alleine in Betracht.

Mit besonderem Vorteil werden als saure Katalysatoren Zeolithe des Pentasiltyps eingesetzt. Diese Zeolithe können unterschiedliche chemische Zusammensetzungen aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische. Beispielsweise handelt es sich bei den Aluminosilikatzeolithen um kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationenin den Kristall, z.B. eines Alkali- oder Wasserstoffions, ausgeglichen. Ein Kationenaustausch ist möglich. Die Räume zwischen den Tetraedern sind vor der Dehydratation durch Trocknen bzw. Calcinieren von Wassermolekeln besetzt. Bei den anderen vorstehend neben den Aluminosilikatzeolithen genannten Zeolithen handelt es sich um kristalline Verbindungen mit Zeolithstruktur, bei denen dreiwertige Elemente wie B, Fe, Ga, Cr, As, Bi anstelle des Aluminiums oder vierwertige Elemente wie Ge anstelle des Siliciums in das Zeolithgitter eingebaut sind.

Insbesondere eignen sich Alumino-, Boro- und Eisensilikatzeolithe des Pentasiltyps als Katalysatoren für das erfindungsgemäße Verfahren.

Die Aluminosilikatzeolithe werden beispielsweise in der Weise hergestellt, daß man eine Aluminiumverbindung, vorzugsweise ein Aluminiumhydroxid wie $Al(OH)_3$, oder ein Aluminiumsalz wie Aluminiumsulfat und eine Siliciumkomponente, zweckmäßig Siliciumdioxid in vorzugsweise hochdisperser Form, in zweckmäßig flüssigem Medium, gegebenenfalls mit Alkali- und/oder Erdalkalizusatz, bei erhöhten Temperaturen, im allgemeinen bei Temperaturen von 100° C bis 220° C, zweckmäßig unter autogenem Druck, miteinander umsetzt. Als flüssiges Medium kommen für die Zeolithherstellung beispielsweise Wasser allein, wäßrige Alkalilauge, Alkohole wie Methanol, Ethanol, Propanol, Butanol, Ethylenglykol, 1,4-Butandiol gegebenenfalls in Mischung mit Wasser, Ether wie Ethylenglykolmonomethylether, Ethylenglykoldimethylether, Diethylenglykolmonomethylether, Diethylenglykoldimethylether gegebenenfalls in Mischung mit Wasser, vorzugsweise Amine wie 1,6-Hexandiamin, 1,3-Propandiamin, Triethylentetramin, zweckmäßig in Mischung mit Wasser, in Betracht. Die erhaltenen Aluminosilikatzeolithe enthalten je nach Wahl der Einsatzstoffmengen ein $SiO_2/Al_2O_3$-Verhältnis von 10 bis 40.000.

Die Borosilikatzeolithe vom Pentasiltyp lassen sich beispielsweise entsprechend der vorstehend beschriebenen Herstellung der Aluminosilikatzeolithe herstellen, indem anstelle der Aluminiumverbindung eine Borverbindung, z.B. $HBO_3$, mit der Siliciumverbindung umgesetzt wird. Dabei werden vorzugsweise Reaktionstemperaturen von 90° C bis 200° C angewendet.

Die Eisensilikatzeolithe vom Pentasiltyp lassen sich beispielsweise ebenso entsprechend der vorstehend beschriebenen Herstellung der Aluminosilikatzeolithe herstellen, indem anstelle der Aluminiumverbindung eine Eisenverbindung, vorzugsweise $Fe_2(SO_4)_3$, mit der Siliciumverbindung umgesetzt wird. Dabei werden vorzugsweise Reaktionstemperaturen von 100° C bis 220° C angewendet.

Die erhaltenen Alumino-, Boro- und Eisensilikatzeolithe werden beispielsweise in der Weise zu dem einzusetzenden Katalysator weiterverarbeitet, daß man sie nach ihrer Isolierung, Trocknung bei Temperaturen von im allgemeinen 80° C bis 160° C, vorzugsweise 100° C bis 120° C, und Calcination bei Temperaturen von zweckmäßig 450° C bis 550° C, vorzugsweise 490° C bis 510° C, zweckmäßig mit einem Bindemittel im Verhältnis von im allgemeinen 90:10 bis 40:60 Gew.% zu z.B. Strängen, Pellets oder Tabletten verformt. Als Bindemittel eignen sich beispielsweise Aluminiumoxide, vorzugsweise Boehmit, Aluminosilikate, zweckmäßig in amorpher Form, mit einem $SiO_2/Al_2O_3$-Verhältnis von im allgemeinen 25:75 bis 95:5, vorzugsweise 1:1 bis 10:1, insbesondere etwa 75:25, Siliciumdioxid, bevorzugt in hochdisperser Form, Gemische aus zweckmäßig hochdispersem Siliciumdioxid und hochdispersem Aluminiumoxid, zweckmäßig hochdisperses Titandioxid und Ton. Nach der Verformung werden die Extrudate oder Preßlinge zweckmäßig getrocknet, in der Regel 10 bis 20 Stunden, z.B. ca. 16 Stunden, im allgemeinen bei Temperaturen von 80° C bis 160° C, vorzugsweise 100° C bis 120° C, und danach calciniert, im allgemeinen für einen Zeitraum von 10 bis 20 Stunden, z.B. 16 Stunden, zweckmäßig bei Temperaturen von 450° C bis 550° C, vorzugsweise 490° C bis 520° C.

Eine weitere Ausführungsform zur Weiterverarbeitung zu dem einzusetzenden Katalysator besteht darin, daß der isolierte Alumino-, Boro- und Eisensilikatzeolith direkt nach der Trocknung verformt wird und erstmals nach der Verformung einer Calcination unterworfen wird.

Die erhaltenen Alumino-, Boro- und Eisensilikatzeolithe können aber auch in reiner Form, d.h. ohne Bindemittel, verformt werden und z.B. als Stränge, Tabletten, Pellets oder Wirbelgut eingesetzt werden.

Liegt der Zeolith von der Synthese her nicht in der katalytisch aktiven, aciden H-Form vor, sondern z.B. in der Na-Form, dann kann diese durch Ionenaustausch mit Ammoniumionen und anschließende Calcination oder durch Behandlung mit Säuren vollkommen oder partiell in die gewünschte H-Form übergeführt werden.

3

Zur Erhöhung der Selektivität, der Standzeit und der Anzahl der möglichen Regenerierungen lassen sich unterschiedliche Modifizierungen an den erfindungsgemäß zu verwendenden zeolithischen Katalysatoren vornehmen.

Eine Art der Modifizierung der Katalysatoren besteht darin, daß man den unverformten oder verformten Zeolithen mit Alkalimetallen wie Na, Erdalkalimetallen wie Ca, Mg, Erdmetallen wie B, Tl und vorzugsweise Übergangsmetallen wie Mn, W, Fe, Mo, Cu, Zn, Edelmetallen wie Pd und/oder Seltenen Erdmetallen wie La, Ce ionenaustauscht oder dotiert. Eine Ausführungsform für diese Mofifizierung besteht beispielsweise darin, daß man den verformten Pentasilzeolithen in einem Steigrohr vorlegt und bei Raumtemperatur oder erhöhten Temperaturen, z.B. bei Temperaturen von 20 °C bis 120 °C, vorzugsweise 20 °C bis 100 °C, ein Salz der voranbeschriebenen Metalle, z.B. ein Halogenid oder ein Nitrat, zweckmäßig in wäßriger Lösung, darüberleitet. Ein derartiger Ionenaustausch kann z.B. an der Wasserstoff-, Ammonium- oder Alkaliform des Zeolithen vorgenommen werden. Eine weitere Ausführungsform für die Modifizierung besteht darin, daß man das zeolithische Material mit einer Verbindung der voranbeschriebenen Metalle, z.B. mit einem Halogenid, Nitrat und/oder Oxid, in flüssigem Medium, z.B. in wäßriger oder alkoholischer Lösung, imprägniert. Sowohl an einen Ionenaustausch als auch an eine Imprägnierung schließt sich zweckmäßig zumindest eine Trocknung und/oder Calcination an. Eine weitere Ausführungsform für die Modifizierung besteht z.B. darin, daß man den reinen, pulverförmigen Zeolithen in einer ammoniakalischen Lösung eines Salzes des aufzutragenden Metalles, z.B. in einer ammoniakalischen $Pd(NO_3)_2$-Lösung, unter Rühren bei Temperaturen von 20 bis 120 °C, vorzugsweise 40 bis 100 °C, über einen Zeitraum von zweckmäßig 6 bis 30 Stunden, z.B. ca. 24 Stunden, aufschlämmt. Nach Abfiltrieren, Trocknen bei im allgemeinen 120 bis 180 °C, vorzugsweise 140 bis 160 °C, und Calcination bei im allgemeinen ca. 450 bis 550 °C, vorzugsweise 480 bis 520 °C, kann das so gewonnene zeolithische Material mit oder ohne Bindemittel zu verformten Teilchen, z.B. zu Strängen oder Pellets, weiterverarbeitet werden. In einer weiteren Ausführungsform für die Modifizierung wird ein Ionenaustausch des in der H-Form vorliegenden Zeolithen z.B. in der Weise durchgeführt, daß man den verformten Zeolithen, z.B. als Stränge oder Pellets, in einer Kolonne vorlegt und eine ammoniakalische Lösung eines Salzes des aufzutragenden Metalles, z.B. eine ammoniakalische Pd-$(NO_3)_2$-Lösung, bei leicht erhöhter Temperatur, im allgemeinen bei Temperaturen von 30 bis 80 °C, im Kreislauf zweckmäßig 10 bis 25 Stunden, vorzugsweise 15 bis 20 Stunden, darüberleitet. Danach wird zweckmäßig mit Wasser ausgewaschen, bei z.B. ca. 150 °C getrocknet und bei beispielsweise 550 °C calciniert. Es kann vorteilhaft sein, bei den metalldotierten Zeolithen eine Nachbehandlung mit Wasserstoff anzuschließen.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder mit Wasserdampf unterwirft. Auch kann durch partielle Verkokung (pre-coke) die Aktivität des Katalysators für ein Selektivitätsoptimum des gewünschten Reaktionsproduktes eingestellt werden.

In einer weiteren Ausführungsform des erfindungsgemäßen Verfahrens wird für die Isomerisierung als saurer Katalysator ein Metallphosphat, insbesondere ein Metallhydrogenphosphat, verwendet. Diese Phosphate können Phosphorsäure auch im Überschuß, der über die stöchiometrische Zusammensetzung der sauren Metallphosphate hinausgeht, enthalten, z.B. in einem Überschuß bis zu 65 %, vorzugsweise bis zu 20 %, insbesondere bis zu 10 %. Als derartige Metallphosphate können beispielsweise Magnesiumphosphate, Calciumphosphate, Strontiumphosphate, Bariumphosphate, Manganphosphate, Nickelphosphate, Kupferphosphate, Kobaltphosphate, Cadmiumphosphate, Eisen(II)-phosphate, Chromphosphate und insbesondere Aluminiumphosphate verwendet werden. Der Metallphosphat-Katalysator kann als solcher oder auf einem Trägermaterial verwendet werden. Geeignete Trägermaterialien sind beispielsweise Aluminiumoxid, Kieselsäure, Aktivkohle und Zinkoxid.

Bei Nachlassen der Aktivität der festen sauren Katalysatoren werden diese zweckmäßig regeneriert. Eine vorteilhafte Methode zur Regenerierung der festen Katalysatoren besteht darin, daß man den Katalysator mit Phosphorsäure, vorzugsweise mit wäßriger Phosphcrsäure behandelt. Diese Behandlung mit Phosphorsäure kann kontinuierlich, d.h. ohne Unterbrechung der Isomerisierungsreaktion durchgeführt werden, indem man zweckmäßig im Bereich der Isomerisierungstemperatur während der Isomerisierung zweckmäßig wäßrige Phosphorsäure auf den Katalysator aufgibt, wobei die Phosphorsäure im allgemeinen in einer solchen Menge zugegeben wird, daß, bezogen auf den Katalysator und berechnet als $P_2O_5$, stündlich $10^{-6}$ bis $10^{-3}$, vorzugsweise $10^{-5}$ bis $10^{-4}$ kg $P_2O_5$ je kg Katalysator zugegeben werden. Die Regenerierung des festen Katalysators kann auch diskontinuierlich, d.h. durch Unterbrechung der Isomerisierungsreaktion, durchgeführt werden. Die diskontinuierliche Isomerisierung erfolgt ebenfalls zweckmäßig bei erhöhter Temperatur, z.B. bei Temperaturen von 50 bis 300 °C, vorzugsweise 50 bis 250 °C, insbesondere 50 bis 150 °C, wobei die Phosphorsäure im allgemeinen in einer solchen Menge auf den Katalysator aufgegeben wird, daß, bezogen auf den Katalysator und berechnet als $P_2O_5$, etwa 0,1 bis 20 Gew.% $P_2O_5$

zugegeben werden. Im allgemeinen erfolgt die Behandlung des festen Katalysators mit Phosphorsäure in der Weise, daß man die Phosphorsäure zweckmäßig als wäßrige Phosphorsäure über den Katalysator rieseln läßt.

Der feste saure Katalysator, insbesondere der zeolithische Katalysator, läßt sich weiter vorteilhaft durch Abbrennen mit Sauerstoff enthaltenden Gasen, vorzugsweise Luft, regenerieren. Es kann vorteilhaft sein, die Luft mit inerten Gasen, z.B. Stickstoff zu verdünnen. Die Behandlung der Katalysatoren mit den Sauerstoff enthaltenden Gasen wird im allgemeinen bei Temperaturen von 300° C bis 600° C, vorzugsweise 400 bis 550° C, insbesondere480 bis 520° C, durchgeführt. Hierdurch kann die Anfangsaktivität der Katalysatoren in einfacher Weise zurückerhalten werden.

Es ist ein Vorteil des erfindungsgemäßen Verfahrens, daß ein Wasserdampfzusatz zu dem in die Isomerisierungszone eingeleiteten, 1-Buten enthaltenden $C_4$-Kohlenwasserstoffgemisch nicht erforderlich ist. Es ist zwar auch möglich, das zu isomerisierende $C_4$-Kohlenwasserstoffgemisch mit Wasserdampf zu verdünnen. Dabei wird man jedoch den Wasserdampfzusatz zu dem zu isomerisierenden $C_4$-Kohlenwasserstoffgemisch in der Regel auf weniger als 1 Mol, vorzugsweise auf höchstens 0,8 Mol, insbesondere auf höchstens 0,5 Mol Wasser je Mol 1-Buten begrenzen.

Die hier beschriebenen Katalysatoren können wahlweise in Form von Strängen, z.B. als 2 bis 4 mm Stränge, von Tabletten, z.B. als Tabletten mit 3 bis 5 mm Durchmesser, als Pulver z.B. mit Teilchengrößen von 0,1 bis 0,5 mm, als Pellets, z.B. mit 2 bis 5 m/m Durchmesser oder als Wirbelkontakt eingesetzt werden.

Die Menge des sauren Katalysators beträgt im allgemeinen etwa 0,01 bis 10 kg, vorzugsweise etwa 0,03 bis 2 kg je kg/h Durchsatz des zu isomerisierenden 1-Butens durch den Reaktor. Vorzugsweise werden für die Isomerisierung Festbettreaktoren verwendet.

Die Isomerisierung des 1-Butens zu 2-Butenen in der Isomerisierungszone wird im allgemeinen bei Temperaturen von 80 bis 400° C, vorzugsweise 100 bis 350° C, insbesondere 150 bis 250° C, durchgeführt. Die Isomerisierung kann bei Atmosphärendruck durchgeführt werden. Vorzugsweise erfolgt die Isomerisierung jedoch bei erhöhtem Druck, zweckmäßig bei Drücken bis zu 100 bar, wobei vorzugsweise Drucke von 2 bis 60 bar, insbesondere 6 bis 40 bar, angewandt werden.

Das aus der Isomerisierungszone erhaltene Isomerisierungsgemisch wird anschließend zur Abtrennung der 2-Butene von dem 1-Buten in die destillative Trennzone geleitet, wobei oberhalb des unteren Drittels der destillativen Trennzone, im allgemeinen in der oberen Hälfte der destillativen Trennzone, eine 1-Buten und zusätzlich 2-Buten-trans enthaltende Fraktion abgezogen wird, die in die Isomerisierungszone zurückgeführt wird. Gegebenenfalls im Isomerisierungsgemisch vorhandenes Isobutan und/oder Isobuten werden zweckmäßig in der oberen Hälfte, vorzugsweise im oberen Drittel, insbesondere am Kopf der destillativen Trennzone abgezogen. Dabei werden für die destillative Trennung im allgemeinen übliche Destillationskolonnen mit beispielsweise ca. 100 praktischen Böden verwendet.

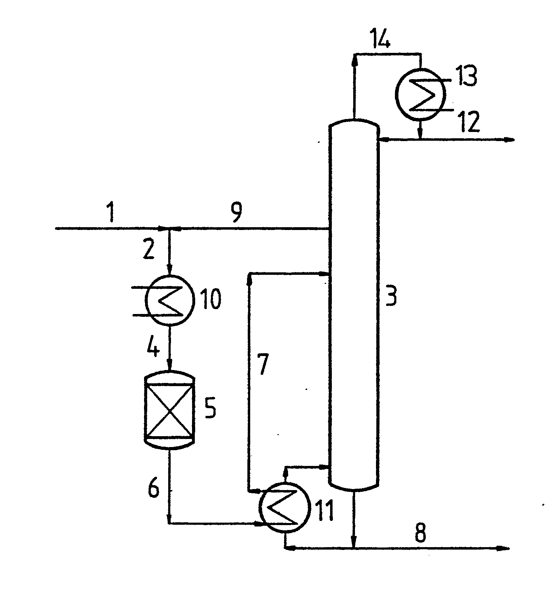

In der Figur wird eine beispielhafte Ausführungsform des erfindungsgemäßen Verfahrens schematisch erläutert. Das 1-Buten, n-Butan und ggf. 2-Butene enthaltende Ausgangs-$C_4$-Kohlenwasserstoffgemisch wird über Leitungen 1,2 und 4 und nach Vorheizung im Wärmetauscher 10 dem Isomerisierungsreaktor 5 zugeführt. Das aus der Isomerisierungszone 5 erhaltene Isomerisierungsgemisch wird über Leitungen 6 und 7 nach Abkühlung im Aufkocher 11 für die Destillationskolonne 3 der Destillationskolonne 3 zugeführt. Über Leitungen 9, 2 und 4 wird aus der Destillationskolonne eine 1-Buten und zusätzlich 2-Buten-trans enthaltende Fraktion abgezogen und in den Isomerisierungsreaktor zurückgeführt, in dem an einem sauren Katalysator die Isomerisierung des 1-Butens zu den 2-Butenen erfolgt.

Am Sumpf der Destillationskolonne 3 werden über Leitung 8 die 2-Butene abgezogen. Im Ausgangs-$C_4$-Kohlenwasserstoffgemisch enthaltenes Isobutan und n-Butan werden nach Durchlaufen des Isomerisierungsreaktors am Kopf der Destillationskolonne 3 über Leitung 12 abgezogen. Der Kopfabzug der Kolonne 3 wird über Leitung 14 abgezogen und dem Kondensator 13 wieder zugeführt. Der verflüssigte Rückfluß wird der Destillationskolonne 3 wieder zugeführt. Das Kopfprodukt wird über Leitung 12 gasförmig oder flüssig entnommen.

2-Butene sind wichtige Ausgangsstoffe beispielsweise für die Herstellung von 2-Methylbutanal.

In der nachstehenden Tabelle werden Umsätze und Selektivitäten, die mit unterschiedlichen Katalysatoren bei der Isomerisierung von 1-Buten zu 2-Buten-cis und 2-Buten-trans erzielt wurden, beschrieben. Die Reaktionen wurden unter isothermen Bedingungen in einem Rohrreaktor (0,6 cm Innendurchmesser, 90 cm Länge) in der Gasphase durchgeführt. Die quantitative und qualitative Bestimmung des Produktionsgemisches erfolgte gaschromatographisch. Die verwendeten Katalysatoren wurden wie folgt hergestellt:

Katalysator A

Der Aluminiumsilikatzeolith des Pentasil-Typs wurde unter hydrothermalen Bedingungen bei autogenem Druck und 150°C aus 65 g hochdispersem $SiO_2$, 20,3 g $Al_2(SO_4)_3$ x 18 $H_2O$ in 1 kg einer 50 gew.%igen wäßrigen 1,6-Hexandiamin-Lösung in einem Rührautoklaven synthetisiert. Nach Abfiltrieren und Auswaschen wurde das kristalline Reaktionsprodukt bei 110°C 24 Stundengetrocknet und bei 500°C 24 Stunden calciniert. Dieser Aluminosilikatzeolith enthielt 91,6 Gew.% $SiO_2$ und 4,6 Gew.% $Al_2O_3$.

Katalysator B

Der Borzeolith des Pentasil-Typs wurde in einer hydrothermalen Synthese aus 64 g hochdispersem $SiO_2$, 12,2 g $H_3BO_3$, in 800 g einer 50 gew.%igen wäßrigen 1,6-Hexandiamin-Lösung bei 170°C unter autogenem Druck in einem Rührautoklaven hergestellt. Nach Abfiltrieren und Auswaschen wurde das kristalline Reaktionsprodukt bei 100°C 24 Stunden getrocknet und bei 500°C 24 Stunden calciniert. Dieser Borosilikatzeolith setzte sich zusammen aus 94,2 Gew.% $SiO_2$ und 2,32 Gew.% $B_2O_3$.

Mit diesem Material wurden durch Verformen mit Boehmit im Gewichtsverhältnis 60:40 2-mm-Stränge hergestellt, die bei 110°C 16 Stunden getrocknet und bei 500°C 24 Stunden calciniert wurden.

Katalysator C

Katalysator C wurde aus einem Eisensilikatzeolith durch Verformung zu Strängen mit Boehmit im Gewichtsverhältnis 60:40 und anschließender Calcination bei 500°C/16 Stunden hergestellt. Der Eisensilikatzeolith des Pentasil-Typs wurde unter hydrothermalen Bedingungen bei autogenem Druck und 165°C aus 273 g Wasserglas, gelöst in 253 g einer 50 gew.%igen wäßrigen 1,6-Hexandiamin-Lösung, und 31 g Eisensulfat, gelöst in 21 g 96 gew.%iger Schwefelsäure und 425 g Wasser, in einem Rührautoklaven während 4 Tagen synthetisiert, danach abfiltriert, ausgewaschen, bei 100°C/24 Stunden getrocknet und bei 500°C/24 Stunden calciniert. Dieser Eisensilikatzeolith hat ein $SiO_2/Fe_2O_3$-Verhältnis von 17,7 und einen $Na_2O$-Gehalt von 0,62 Gew.%.

Katalysator D

Über Katalysator B wurde eine ammoniakalische $Pd(NH_3)_4(NO_3)_2$-Lösung im Kreis gepumpt (65 ml/min). Danach wurde bei 110°C getrocknet und bei 500°C calciniert. Der Pd-Gehalt des erhaltenen Katalysators betrug 3,3 Gew.%.

Katalysator E

Katalysator B wurde bei 80°C mit einer 20 gew.%igen NaCl-Lösung ionenausgetauscht. Nach der Calcination bei 500°C enthielt der erhaltene Katalysator 0,28 Gew.% Na.

Katalysator F

Katalysator B wurde mit Mg-Acetat ca. 30 min getränkt, bei 130°C getrocknet und 540°C calciniert. Der Mg-Gehalt des erhaltenen Katalysators betrug 1,88 Gew.%.

Katalysator G

Katalysator B wurde mit $Ba(OH)_2$ x 8 $H_2O$ ca. 30 min getränkt, bei 130°C getrocknet und bei 540°C calciniert. Der Ba-Gehalt des erhaltenen Katalysators betrug 3,1 Gew.%.

Katalysator H

Katalysator B wurde mit $Zn(NO_3)_2$ x 6 $H_2O$ ca. 30 min getränkt, bei 130°C getrocknet und bei 540°C calciniert. Der Zn-Gehalt des erhaltenen Katalysators betrug 3,4 Gew.%.

Katalysator I

Katalysator B wurde mit $Cd(NO)_3$ x 4 $H_2O$ ca. 30 min getränkt, bei 130° C getrocknet und bei 540° C calciniert. Der Cd-Gehalt des erhaltenen Katalysators betrug 6,4 Gew.%.

Katalysator J

Katalysator B wurde mit $Fe(NO)_3$ x 9 $H_2O$ ca. 30 min getränkt, bei 130° C getrocknet und bei 540° C calciniert. Der Fe-Gehalt des erhaltenen Katalysators betrug 3,3 Gew.%.

Katalysator K

Katalysator B wurde mit $Co(NO)_3$ x 6 $H_2O$ ca. 30 min getränkt, bei 130° C getrocknet und bei 540° C calciniert. Der Co-Gehalt des erhaltenen Katalysators betrug 3,8 Gew.%.

Katalysator L

Katalysator B wurde mit $Ni(NO)_3$ x 6 $H_2O$ ca. 30 min getränkt, bei 130° C getrocknet und bei 540° C calciniert. Der Ni-Gehalt des erhaltenen Katalysators betrug 3,7 Gew.%.

## Tabelle

Isomerisierung von 1-Buten zu 2-Buten bei einer Reaktionstemperatur von 200°C und einer Belastung (WHSV) des verwendeten Katalysators von $1\ h^{-1}$

| Katalysator | A | B | C | D | E | F | G | H | I | J | K | L |
|---|---|---|---|---|---|---|---|---|---|---|---|---|
| Produktzusammensetzung Gew.% | | | | | | | | | | | | |
| 1-Buten | 15,7 | 14,8 | 25,0 | 14,54 | 50,4 | 20,51 | 17,42 | 17,28 | 23,03 | 15,06 | 15,72 | 14,75 |
| 2-Buten-cis | 33,2 | 33,4 | 31,1 | 31,33 | 20,4 | 29,96 | 30,81 | 30,51 | 29,76 | 30,74 | 30,54 | 31,42 |
| 2-Buten-trans | 50,5 | 51,4 | 43,4 | 53,65 | 28,7 | 48,94 | 51,21 | 51,48 | 46,8 | 52,98 | 52,41 | 52,98 |

In der Figur wird der Einfluß der Temperatur auf die Zusammensetzung des Reaktionsaustrages bei Verwendung von Katalysator B bei einer Belastung des Katalysators von 1 h$^{-1}$ demonstriert. Es bedeuten in der Figur:

```
C   = Konzentration in Gew.%
T   = Temperatur in °C
B2T = 2-Buten-trans
B2C = 2-Buten-cis
B1  = 1-Buten
```

## Ansprüche

1. Verfahren zur Gewinnung von 2-Butenen aus 1-Buten, n-Butan und gegebenenfalls 2-Butene enthaltenden C$_4$-Kohlenwasserstoffgemischen, bei dem man das 1-Buten, n-Butan und gegebenenfalls 2-Butene enthaltende C$_4$-Kohlenwasserstoffgemisch einer Isomerisierungszone (5) zuführt (1, 2, 4), in der Isomerisierungszone das 1-Buten bei erhöhten Temperaturen zu 2-Butenen isomerisiert, das aus der Isomerisierungszone erhaltene Isomerisierungsgemisch in das untere oder mittlere Drittel der destillativen Trennzone (3) leitet (7), oberhalb des unteren Drittels der destillativen Trennzone eine 1-Buten und zusätzlich 2-Buten-trans enthaltende Fraktion abzieht (9) und in die Isomerisierungszone leitet (2, 4), im unteren Drittel der destillativen Trennzone die 2-Butene bzw. eine die 2-Butene enthaltende Fraktion abzieht (8) und gleichzeitig eine n-Butan enthaltende Fraktion aus der destillativen Trennzone an einem Punkt oberhalb des unteren Drittels der destillativen Trennzone abzieht (12).

2. Verfahren nach Anspruch 1, dadurch gekennzeichnet, daß man eine solche Menge an 2-Buten-trans in die Isomerisierungszone zurückführt, daß das Gewichtsverhältnis von in die Isomerisierungszone

EP 0 170 182 B1

zurückgeführtem 2-Buten-trans zu dem abgezogenen n-Butan 100 : 1 bis 1 : 100 beträgt.

**3.** Verfahren nach Anspruch 1 und 2, dadurch gekennzeichnet, daß für die Isomerisierung saure Katalysatoren eingesetzt werden.

**4.** Verfahren nach Anspruch 1 bis 3, dadurch gekennzeichnet, daß man als Katalysatoren Phosphorsäuren und/oder Phosphate enthaltende Katalysatoren verwendet.

**5.** Verfahren nach Anspruch 1 bis 3, dadurch gekennzeichnet, daß man als Katalysatoren Zeolithe des Pentasil-Typs enthaltende Katalysatoren verwendet.

**6.** Verfahren nach Anspruch 1 bis 3, dadurch gekennzeichnet, daß man Aluminosilikatzeolithe enthaltende Katalysatoren verwendet.

**7.** Verfahren nach Anspruch 1 bis 3 und 5, dadurch gekennzeichnet, daß man Borosilikatzeolithe enthaltende Katalysatoren verwendet.

**8.** Verfahren nach Anspruch 1 bis 3 und 5, dadurch gekennzeichnet, daß man Eisensilikatzeolithe enthaltende Katalysatoren verwendet.

**9.** Verfahren nach Anspruch 1 bis 3 und 5 bis 8, dadurch gekennzeichnet, daß man mit Übergangsmetallen dotierte Zeolithe als Katalysatoren verwendet.

**10.** Verfahren nach Anspruch 1 bis 3 und 5 bis 8, dadurch gekennzeichnet, daß man mit Edelmetallen dotierte Zeolithe als Katalysatoren verwendet.

**11.** Verfahren nach Anspruch 1 bis 3 und 5 bis 8, dadurch gekennzeichnet, daß man mit seltenen Erdmetallen dotierte Katalysatoren verwendet.

**12.** Verfahren nach Anspruch 10, dadurch gekennzeichnet, daß man Palladium-dotierte Zeolithe als Katalysatoren verwendet.

**13.** Verfahren nach Anspruch 1 bis 12, dadurch gekennzeichnet, daß man die Isomerisierung in Gegenwart von Wasserstoff durchführt.

## Claims

**1.** A process for obtaining but-2-enes from a $C_4$-hydrocarbon mixture which contains but-1-ene and n-butane and may or may not contain but-2-enes, the said hydrocarbon mixture being fed (1, 2, 4) to an isomerization zone (5), the but-1-ene is isomerized to but-2-enes at elevated temperatures in the isomerization zone, the isomerization mixture obtained from the isomerization zone is passed (7) into the lower or middle third of the distillative separation zone (3), a fraction containing but-1-ene and additionally trans-but-2-ene is removed (9) above the lower third of the distillative separation zone and is passed (2, 4) into the isomerization zone and the but-2-enes or a fraction containing the but-2-enes are or is removed (8) from the lower third of the distillative separation zone and at the same time a fraction containing n-butane is removed (12) from the distillative separation zone at a point above the lower third of the distillative separation zone.

**2.** A process as claimed in claim 1, wherein trans-but-2-ene is recycled to the isomerization zone in an amount such that the weight ratio of trans-but-2-ene recycled to the isomerization zone to the n-butane removed is from 100:1 to 1:100.

**3.** A process as claimed in claim 1 or 2, wherein an acidic catalyst is employed for the isomerization.

**4.** A process as claimed in any of claims 1 to 3, wherein catalysts containing phosphoric acids and/or phosphates are used as catalysts.

10

5. A process as claimed in any of claims 1 to 3, wherein the catalyst used is one which contains a zeolite of the pentasil type.

6. A process as claimed in any of claims 1 to 3, wherein a catalyst containing an aluminosilicate zeolite is used.

7. A process as claimed in any of claims 1 to 3 or 5, wherein a catalyst containing a borosilicate zeolite is used.

8. A process as claimed in any of claims 1 to 3 or 5, wherein a catalyst containing an iron silicate zeolite is used.

9. A process as claimed in any of claims 1 to 3 and 5 to 8, wherein the catalyst used is a zeolite doped with transition metals.

10. A process as claimed in any of claims 1 to 3 and 5 to 8, wherein the catalyst used is a zeolite doped with noble metals.

11. A process as claimed in any of claims 1 to 3 and 5 to 8, wherein a catalyst doped with rare earth metals is used.

12. A process as claimed in claim 10, wherein a palladium-doped zeolite is used as the catalyst.

13. A process as claimed in any of claims 1 to 12, wherein the isomerization is carried out in the presence of hydrogen.


**Revendications**

1. Procédé pour l'obtention de 2-butènes à partir de mélanges d'hydrocarbures en C₄ qui contiennent du 1-butène, du n-butane et éventuellement des 2-butènes, dans lequel on introduit (1, 2, 4) dans une zone d'isomérisation (5) le mélange d'hydrocarbures en C₄ contenant du 1-butène, du n-butane et éventuellement des 2-butènes, on isomérise le 1-butène en 2-butènes à température élevée dans la zone d'isomérisation, on envoie (7) dans le tiers inférieur ou moyen de la zone de séparation par distillation (3) le mélange d'isomérisation obtenu dans la zone d'isomérisation, on extrait (9) au-dessus du tiers inférieur de la zone de séparation par distillation une fraction contenant du butène-1 et en outre du trans-2-butène et on l'envoie (2, 4) dans la zone d'isomérisation, on extrait (8) dans le tiers inférieur de la zone de séparation par distillation les 2-butènes ou une fraction contenant les 2-butènes et, en même temps, on extrait (12) une fraction contenant du n-butane de la zone de séparation par distillation en un point situé au-dessus du tiers inférieur de la zone de séparation par distillation.

2. Procédé selon la revendication 1, caractérisé en ce qu'on renvoie dans la zone d'isomérisation une quantité de trans-2-butène telle que le rapport en poids du trans-2-butène renvoyé dans la zone d'isomérisation au n-butane extrait se situe entre 100:1 à 1:100.

3. Procédé selon la revendication 1 ou 2, caractérisé en ce qu'on utilise, pour l'isomérisation, des catalyseurs acides.

4. Procédé selon l'une quelconque des revendications 1 à 3, caractérisé en ce qu'on utilise, comme catalyseurs, des catalyseurs contenant des acides phosphoriques et/ou des phosphates.

5. Procédé selon l'une quelconque des revendications 1 à 3, caractérisé en ce qu'on utilise, comme catalyseurs, des catalyseurs contenant des zéolithes du type pentasile.

6. Procédé selon l'une quelconque des revendications 1 à 3, caractérisé en ce qu'on utilise des catalyseurs contenant des zéolithes d'aluminosilicate.

7. Procédé selon l'une quelconque des revendications 1 à 3 et 5, caractérisé en ce qu'on utilise des catalyseurs contenant des zéolithes de borosilicate.

8. Procédé selon l'une quelconque des revendications 1 à 3 et 5, caractérisé en ce qu'on utilise des catalyseurs contenant des zéolithes de ferrosilicate.

9. Procédé selon l'une quelconque des revendications 1 à 3 et 5 à 8, caractérisé en ce qu'on utilise, comme catalyseurs, des zéolithes dopées avec des métaux de transition.

10. Procédé selon l'une quelconque des revendications 1 à 3 et 5 à 8, caractérisé en ce qu'on utilise, comme catalyseurs, des zéolithes dopées avec des métaux précieux.

11. Procédé selon l'une quelconque des revendications 1 à 3 et 5 à 8, caractérisé en ce qu'on utilise des zéolithes dopées avec des métaux des terres rares.

12. Procédé selon la revendication 10, caractérisé en ce qu'on utilise, comme catalyseurs, des zéolithes dopées avec du palladium.

13. Procédé selon l'une quelconque des revendications 1 à 12, caractérisé en ce qu'on effectue l'isomérisation en présence d'hydrogène.